(19) **Europäisches Patentamt**

**European Patent Office**

**Office européen des brevets**

(11) Publication number: **0 136 802 B1**

(12) **EUROPEAN PATENT SPECIFICATION**

(45) Date of publication of patent specification: **20.03.91**

(51) Int. Cl.⁵: **C12P 7/18**, //(C12P7/18, C12R1:645)

(21) Application number: **84305746.4**

(22) Date of filing: **22.08.84**

(54) Industrial-scale process for the production of polyols by fermentation of sugars (1111111).

(30) Priority: **24.08.83 GB 8322750**
**11.10.83 GB 8327193**

(43) Date of publication of application:
**10.04.85 Bulletin 85/15**

(45) Publication of the grant of the patent:
**20.03.91 Bulletin 91/12**

(84) Designated Contracting States:
**AT BE CH DE FR GB IT LI NL SE**

(56) References cited:

ARCH. MICROBIOL., vol. 98, 1974, pages 339-349, Springer-Verlag; L. HANSSENS et al.: "Cyanide-insensitive respiration in Moniliella tomentosa and the effect of drugs on respiration and polyol biosynthesis"

APPLIED AND ENVIRONMENTAL MICROBIOLOGY, vol. 32, no. 1, July 1976, pages 56-63, American Society for Microbiology, US; L. HANSSENS et al.: "Types of respiratory activity in Moniliella tomentosa during growth under different conditions"

(73) Proprietor: **CPC INTERNATIONAL INC.**
**International Plaza P.O. Box 8000**
**Englewood Cliffs New Jersey 07632(US)**

(72) Inventor: **de Troostenbergh, Jean-Claude**
**Cleerbeekmolen 305**
**B-3215 Houwaart(BE)**
Inventor: **Avalosse, Bernard Léon Henri Marie**
**74, rue du Colmy**
**B-5544 Agimont(BE)**

(74) Representative: **Pennant, Pyers et al**
**Stevens, Hewlett & Perkins 5 Quality Court**
**Chancery Lane**
**London, WC2A 1HZ(GB)**

## Description

It is known that aerobic fermentation of a suitable sugar by the yeast-like fungus Moniliella tomentosa var. pollinis will produce erythritol; this was first re-ported by G.J. Hajny, J. H. Smith and J.C. Garver in Applied Microbiology 12, pp 240-246 (May 1964), who designated the microbe as Torula I₂A. Later, in Antonie van Leeuwenboek 37, PP 107-118 (1971), L. Dooms, G.L. Hennebert and H. Verachtert classified the fungus as Moniliella tomentosa var. pollinis , and confirmed its ability to produce erythritol; the authors also set forth a complete morphological description of the microbe which is incorporated herein by reference. In addition, they recognised that Moniliella can exist under two forms, yeast- and mould-like forms, according, essentially, to the applied culture conditions; on page 110 they state, "On agar slants both forms are produced. In stationary liquid media the mould-like form with abundant mycelium and more arthrospores than blastospores develops.

In shake-flask cultures, the yeast-like form predominates with rounded, oval and rectangular cells produced by budding and by fission while no mycelium is formed".

Prior art workers have reported the production of glycerol, erythritol and arabitol from the fermentation of sugars by Moniliella tomentosa var. pollinis but we have found in our industrial development of the process that the product of the fermentation comprises glycerol, erythritol and ribitol in varying amounts. Glycerol is a well known article of commerce but erythritol and ribitol have hitherto not been available on a significant industrial scale. Both of these products have interesting properties as chemical intermediates, their polyhydroxyl functionality indicating a number of uses e.g. as raw material in the production of polyurethane and other polymers. It is obviously of value, therefore, to be able to influence the fermentation reaction so as to produce more erythritol and ribitol than glycerol because the latter is readily available from other sources. In addition, from the theoretical course of the fermentation through the hexose monophosphate pathway, more of the sugar fermented is capable of conversion to ribitol (83%) and erythritol (66%) than to glycerol (50%). We have now found means of influencing the distribution of the polyol components of the fermentation in the direction of increased ribitol production and at the same time of increasing the total polyol yield by controlling the degree of aeration of the fermentation medium. Furthermore, an additional increase in polyol yield may be obtained by recycling cells from a previous fermentation to the fermentation medium.

It has already been reported by Hajny that, at low aeration rates, erythritol is predominently produced together with ethanol. We have now found that, at higher aeration rates, ethanol production is minimised but, moreover, that the proportion of ribitol in the product increases substantially (from less than 2% to up to 20% or more). A higher oxygen transfer rate, therefore, provides a means of optimising sugar conversion into the most economically attractive polyols. By varying the oxygen transfer rate different polyol proportions can be obtained in the polyol product as desired for different applications.

Another means of increasing the conversion yield of the fermentation is to recycle the biomass, i.e. to use the cells which have been removed from one fermentation as inoculum for the next fermentation.

According to the present invention, therefore, an industrial process for producing a polyol mixture comprising glycerol and erythritol by the aerobic fermentation of a suitable sugar by Moniliella tomentosa var. pollinis is characterised in that ribitol is also produced and that the proportion of ribitol in the polyol mixture and the total polyol yield are increased by increasing the degree of aeration of the fermentation medium.

In addition, a further increase in polyol yield may be achieved by recycling of cells from a previous fermentation.

The "degree of aeration" is a combined factor made up of the amount of oxygen fed to the reaction medium combined with the extent of agitation of the medium e.g. as indicated by the speed of an impeller or the speed of rotation of a stirrer. In general, for a given size of fermentation vessel, increase in the degree of agitation of the contents of the vessel for a given volume of oxygen fed will increase the amount of ribitol which is formed. Similarly, at constant agitation, an increase in the volume of oxygen fed will achieve the same effect. Clearly, however, there will be an optimum extent of agitation for a given volume of oxygen fed to the fermentation vessel and vice versa and the optimum conditions may be determined by simple experiment, always bearing in mind that, up to the optimum, an increase in oxygen fed or degree of agitation will favour the desired production of ribitol. We have also found that the optimum will vary according to the size of the fermentation vessel and, in general, the degree of agitation may be less for a given rate of oxygen fed to a larger than to a smaller volume fermentation vessel.

Preferably, the oxygen is supplied as air or as an oxygen/inert gas composition approximating to that of air at a rate in the range 0.1 to 1.5 preferably 0.5 to 1.5 litre/litre of fermentation medium/minute, the degree of agitation being chosen to be an optimum for an air flow within this range for a given size of fermentation vessel.

By "recycling cells from a previous fermentation", we mean that part or all of the cells used for initiating a fermentation are derived from a previous fermentation. The percentage of recycled biomass can be varied from 5% to 100% of that of the previous fermentation. A nitrogen source, as hereinafter described may also be added in various amounts to the fermentation medium depending on the amount of recycled biomass. When 100% of the cells are recycled, the nitrogen source can be omitted so that the cells are fermenting only sugar, thereby reducing significantly the costs of working-up the fermentation medium. In every case the percentage of recycled cells and the amounts of nitrogen source added should be selected so as to ensure an effective fermenting power from one culture to another.

By operating the process according to the present invention the mixture of polyols produced in the fermentation, i.e. glycerol, erythritol and ribitol may contain more than 20% by weight ribitol.

Moniliella tomentosa var. pollinis is available at the Centraal Bureau voor Schmimmelcultures (CBS), Baarn, The Netherlands, as CBS 461.67, and was also deposited and is available to the public at the Commonwealth Mycological Institute Culture Collection (CMI cc) Ferry Lane, Kew, Richmond, Surrey TW9 3AF, United Kingdom, under the number (CMI cc) 271648. The microbial cells are cultivated on a solid medium containing malt extract (4%), yeast extract (0.2%) and agar (2%). The culture so formed is then inoculated to a sterilised medium containing sugar (the amount will be discussed later) and a nitrogen source.

The fermentation is conducted at a temperature of between 27°C and 32°C at a starting pH of between 3 and 6, preferably 4 to 5. The fermentation is stopped when the sugar has been consumed. The amount of sugar contained in the medium can be between 20% and 45%, preferably 30% to 35%. Suitable sugars for use in both the culture and the fermentation are dextrose (glucose), sucrose, fructose or maltose. (Throughout the specification and claims all percentages and parts are by volume unless otherwise stated).

In the prior art process, as described by Hajny, various sources of nitrogen were described for use in the fermentation, for example, yeast extract, urea, corn steep liquor, malt sprouts, black strap molasses, malt extract and distillers dry solubles. In the process of the present invention good results are achieved with 0.5% yeast extract plus 0.1% urea or with 2% corn steep liquor plus 0.02% urea. The starting pH should be between about 3.0 and 6.0 which decreases to about 2.0 to 3.5 during the fermentation. According to L. Hanssens, A. Van Regenmortel and H. Verachtert, Applied Microbiology, Vol. 24, No. 5, pp 831-833 (November 1972), laboratory fermentations held at different constant pH's result in substantial differences in yields of total polyols and erythritol. We have found that, when working with larger scale fermentations (2-litre fermentors or larger) the total polyol and erythritol yields are less sensitive to starting pH within the 4.0 to 6.0 range. In order to avoid or minimise problems of contamination, a starting pH of 4 to 5 is preferred. The fermentation is conducted until all of the sugar has been consumed (fermentation times will normally be between 4 and 12 days, depending upon the amount of sugar used), after which the culture is stopped and the cells are removed from the culture broth, e.g. by centrifuging. The cell-free culture broth which contains erythritol, ribitol and glycerol can be used as such with or without refining (e.g. by ultrafiltration and demineralisation), for certain applications, e.g. in the polymer industry. The refined culture broth can also be concentrated to 60% to 80% dissolved solids and the erythritol cyrstallised therefrom e.g. by the technique described by J.M. Roxburg, J.F.T. Spencer and H.R. Sallens, Canadian Journal of Technology, Vol. 34 pp 248-253 (1956). The liquor remaining after recovery of the erythritol crystals which is a mixture of (non-crystallised) erythritol, ribitol and glycerol, can also be used after appropriate treatment.

The process according to the invention is advantageously carried out in the presence of the polysaccharide Xanthan gum which has the property of retaining the cells of the Moniliella tomentosa var. pollinis in the fermentation medium and reducing their loss via the foam which occurs during the fermentation. Preferably 100 to 500 ppm Xanthan gum may be present and excellent results are obtained with about 300 ppm. More gum may be used than is necessary for retaining the cells in the fermentation medium (i.e. more than about 500 ppm) but such excess is wasteful.

The overall process is also improved by including in the fermentation medium a conventional antifoam agent. Synthetic antifoam agents (e.g. silicone type or fatty alcohols) are preferred over the natural (e.g. lard oil) products because they can be used in smaller quantities and the final fermentation broth does not need extensive refining for their removal. With most synthetic commercial antifoam agents, 200-300 or 400 ppm is sufficient for optimum foam control.

The following examples are intended to illustrate the practice of the invention.

Preparation of the seed culture

500 ml erlenmeyers containing 50 ml culture medium comprising 20% dextrose, 0.5% yeast extract, 0.1% urea and 300 ppm Xanthan gum were inoculated with a colony from a solid medium and cultivation was conducted for 3 to 4 days at a starting pH of 5, a temperature of 30°C and under reciprocating agitation of 100 oscillations/ minute. These cultures were used in the subsequent examples.

Example 1

Six 2-litre fermentors each containing 1.5 litre culture medium comprising 32% dextrose, 0.5% yeast extract, 0.1% urea, 300 ppm Xanthan gum and 300 ppm SAG 471 (a dimethyl-polysiloxane antifoam from Union Carbide) were inoculated with the seed culture in an amount of 2% of the volume of the fermentation medium. The air flow rate was 0.6 litre air per litre fermentation medium/minute.

Different impeller speeds were set in the fermentor varying from 400 rpm to 740 rpm. The temperature was kept constant at 30°C and the fermentation was allowed to proceed for 11 days. At the end of that time, the compositions of the fermentation media were analysed and the polyol yields and compositions were calculated. The results are presented in Table 1.

## Table 1

| Impeller speed rpm | Erythritol | | Glycerol | | Ribitol | |
|---|---|---|---|---|---|---|
| | Yield %(1) | % (2) | Yield %(1) | % (2) | Yield %(1) | % (2) |
| 400 | 28.5 | 87 | 3.8 | 12 | 0.4 | 1 |
| 485 | 28 | 89 | 3.2 | 10 | 0.3 | 1 |
| 535 | 30.7 | 88 | 3.5 | 10 | 0.5 | 2 |
| 620 | 36.8 | 80 | 6.5 | 14 | 2.4 | 6 |
| 665 | 35.8 | 70 | 10.0 | 20 | 5.2 | 10 |
| 740 | 29.6 | 56 | 13.7 | 26 | 9.1 | 17 |

(1) based on dextrose consumed

(2) based on total polyol

Example 2

Five 2-litre fermentors each containing 1.5 litre culture medium comprising 32% dextrose, 300 ppm Xanthan gum and 300 ppm SAG 471 were inoculated with cells recovered from a previous fermentation by centrifugation. The amount of recycled cells was varied from 7% to 100% and the nitrogen source was varied from 0.5% to 0%, i.e. inversely with respect to the quantity of recycled cells. The air flow rate was 0.6 litre/litre/minute and the impeller speed was 680 rpm. The starting pH was adjusted to 4 with dilute hydrochloric acid. The compositions of the fermentation media were analysed after 9 days and the yields calculated. The results are presented in Table 2.

## Table 2

| Cell Recycle % | Erythritol Yield (1) % | Total Polyol Yield (1) % |
|---|---|---|
| 7 | 35 | 43 |
| 20 | 40 | 47 |
| 40 | 38 | 55 |
| 60 | 38 | 55 |
| 100 | 50 | 70 |

(1) based on dextrose consumed

"Total polyol" includes erythritol, glycerol and ribitol.

**Claims**

1. An industrial process for producing a polyol mixture comprising glycerol and erythritol by the aerobic fermentation of a suitable sugar by Moniliella tomentosa var. pollinis is characterised in that ribitol is also produced and that the proportion of ribitol in the polyol mixture and the total polyol yield are increased by increasing the degree of aeration of the fermentation medium.

2. A process according to Claim 1 characterised in that the air or an oxygen/inert gas composition approximating to that of air is supplied at a rate of 0.1 to 1.5 litre/litre of fermentation medium/minute.

3. A process according to Claim 1 or Claim 2 characterised in that cells of the Moniliella tomentosa var. pollinis are recycled from a previous fermentation.

4. A process according to Claim 3 characterised in that 5% to 100% of the cells from a previous fermentation are recycled to the process.

5. The process of any one of the preceding claims characterised in that the sugar is dextrose.

6. The process of any one of the preceding claims characterised in that the sugar is present in the fermentation broth at the beginning of the fermentation in an amount of between 20% and 45%.

7. The process of any one of the preceding claims characterised in that the pH at the beginning of the fermentation is adjusted to between 3 and 6, preferably between 4 and 5.

8. The process of any one of the preceding claims characterised in that Xanthan gum is present in an amount between 100 ppm and 500 ppm of the fermentation medium.

9. The process of any one of the preceding claims characterised in that a conventional antifoam agent is also added to the fermentation.

10. A cell-free, polyol-containing culture broth obtained by fermenting a sugar with Moniliella tomentosa var. pollinis, removing the cells from tbe broth and optionally refining the broth, characterised in that the polyols contained therein consist solely of ribitol, glycerol and erythritol in the following proportions based on total polyols : ribitol, 1%-20%; glycerol, 5%-40%; the balance being erythritol.

11. A cell-free, polyol-containing culture broth obtained by fermenting a sugar with Moniliella tomentosa var. pollinis to form a polyol mixture rich in erythritol, removing the cells, refining and concentrating the broth, crystallising erythritol and removing the erythritol crystals therefrom, characterised in that the polyols contained in the liquor after the crystallisation comprise ribitol, glycerol and erythritol in the following proportions based on total polyols : ribitol, 5%-30%; glycerol, 30%-60%; the balance being erythritol.

**Revendications**

1. Procédé industriel pour la production d'un mélange de polyols comprenant du glycérol et de l'érythritol par fermentation aérobie d'un sucre convenable par Moniliella tomentosa var. pollinis, caractérisé en ce que du ribitol est également produit et en ce que la proportion de ribitol dans le mélange de polyols et le rendement total en polyols sont accrus en augmentant le degré d'aération du milieu de fermentation.

2. Procédé suivant la revendication 1, caractérisé en ce que l'air, ou un mélange oxygène/gaz inerte ayant une composition proche de celle de l'air, est fourni à une vitesse de 0,1 à 1,5 litre/litre de milieu de fermentation/minute.

3. Procédé suivant la revendication 1 ou la revendication 2, caractérisé en ce que les cellules de Moniliella tomentosa var. pollinis provenant d'une fermentation antérieure sont recyclées.

4. Procédé suivant la revendication 3, caractérisé en ce que 5 % à 100 % des cellules provenant d'une fermentation antérieure sont recyclés dans le procédé.

5. Procédé suivant l'une quelconque des revendications précédentes, caractérisé en ce que le sucre est le dextrose.

6. Procédé suivant l'une quelconque des revendications précédentes, caractérisé en ce que le sucre est présent dans le bouillon de fermentation au début de la fermentation en une quantité de 20 % à 45 %.

7. Procédé suivant l'une quelconque des revendications précédentes, caractérisé en ce que le pH au début de la fermentation est ajusté à une valeur comprise dans l'intervalle de 3 à 6, de préférence de 4 à 5.

8. Procédé suivant l'une quelconque des revendications précédentes, caractérisé en ce que de la gomme xanthane est présente en une quantité comprise dans l'intervalle de 100 ppm à 500 ppm du milieu de fermentation.

9. Procédé suivant l'une quelconque des revendications précédentes, caractérisé en ce qu'un agent antimousse usuel est également ajouté au milieu de fermentation.

10. Bouillon de culture dépourvu de cellules, contenant des polyols, obtenu par fermentation d'un sucre avec Moniliella tomentosa var. pollinis , séparation des cellules du bouillon et, facultativement, raffinage du bouillon, caractérisé en ce que les polyols qui y sont présents consistent uniquement en ribitol, glycérol et érythritol en les proportions suivantes, sur la base de la quantité totale de polyols : 1 % à 20 % de ribitol, 5 % à 40 % de glycérol, le reste étant constitué d'érythritol.

11. Bouillon de culture dépourvu de cellules, contenant des polyols, obtenu par fermentation d'un sucre avec Moniliella tomentosa var. pollinis pour former un mélange de polyols riche en érythritol, séparation des cellules, raffinage et concentration du bouillon, cristallisation de l'érythritol et séparation des cristaux d'érythritol de ce bouillon, caractérisé en ce que les polyols présents dans la liqueur après cristallisation consistent en ribitol, glycérol et érythritol en les proportions suivantes, sur la base de la quantité totale de polyols : 5 % à 30 % de ribitol, 30 % à 60 % de glycérol, le reste étant constitué d'érythritol.

**Ansprüche**

1.  Industrielles Verfahren zur Herstellung einer Polyolmischung, enthaltend Glycerin und Erythrit, durch aerobe Gärung eines geeigneten Zuckers mit Moniliella tomentosa var. pollinis, dadurch gekennzeichnet, daß auch Ribit hergestellt wird und daß der Anteil von Ribit in der Polyolmischung und die Ausbeute an Gesamtpolyolen durch Steigerung des Belüftungsgrades des Gärmediums gesteigert wird.

2.  Verfahren nach Anspruch 1, dadurch gekennzeichnet, daß Luft oder ein Sauerstoff/Inertgas-Zusammensetzung, die derjenigen von Luft nahe kommt, in einer Menge im Bereich von 0.1 bis 1.5 Liter/Liter Gärmedium/Minute zugeführt wird.

3.  Verfahren nach Anspruch 1 oder Anspruch 2, dadurch gekennzeichnet, daß Zellen von Moniliella tomentosa var. pollinis aus einer vorhergehenden Gärung rückgeführt werden.

4.  Verfahren nach Anspruch 3, dadurch gekennzeichnet, daß 5% bis 100% der Zellen aus einer vorhergehenden Gärung in das Verfahren rückgeführt werden.

5.  Verfahren nach einem der vorhergehenden Ansprüche, dadurch gekennzeichnet, daß der Zucker Dextrose ist.

6.  Verfahren nach einem der vorhergehenden Ansprüche, dadurch gekennzeichnet, daß der Zucker in der Gärbrühe am Anfang der Gärung in einer Menge zwischen 20% und 45% vorhanden ist.

7.  Verfahren nach einem der vorhergehenden Ansprüche, dadurch gekennzeichnet, daß der pH am Anfang der Gärung zwischen 3 und 6, vorzugsweise zwischen 4 und 5, eingestellt wird.

8.  Verfahren nach einem der vorhergehenden Ansprüche, dadurch gekennzeichnet, daß Xanthangummi in einer Menge zwischen 100 ppm und 500 ppm des Gärmediums vorhanden ist.

9.  Verfahren nach einem der vorhergehenden Ansprüche, dadurch gekennzeichnet, daß auch ein übliches Antischaummittel zur Gärung zugesetzt wird.

10. Zellfreie, Polyol-enthaltende Gärbrühe, erhalten durch Gärung eines Zuckers mit Moniliella tomentosa var. pollinis, Entfernen der Zellen aus der Brühe und gegebenenfalls Reinigung der Brühe, dadurch gekennzeichnet, daß die darin enthaltenen Polyole nur aus Ribit, Glycerin und Erythrit in den folgenden Verhältnissen auf Basis der Gesamtpolyole bestehen: Ribit, 1%-20%; Glycerin, 5%-40%; der Rest ist Erythrit.

11. Zellfreie, Polyol-enthaltende Kulturbrühe, erhalten durch Gärung eines Zuckers mit Moniliella tomentosa var. pollinis unter Bildung einer Polyolmischung, die reich an Erythrit ist, Entfernung der Zellen, Reinigung und Einengen der Brühe, Kristallisieren von Erythrit und Entfernen der Erythritkristalle daraus, dadurch gekennzeichnet, daß die nach der Kristallisation in der Lösung enthaltenen Polyole Ribit, Glycerin und Erythrit in den folgenden Verhältnissen auf Basis der Gesamtpolyole enthalten: Ribit, 5%-30%; Glycerin, 30%-60%, der Rest ist Erythrit.